# EUROPEAN PATENT APPLICATION

(11) **EP 4 817 639 A1**
(43) Date of publication of application: **07.10.2026**
(21) Application number: 25714773.6
(22) Date of filing: 26.02.2025
(51) Int. Cl.: A61L 9/20, A61L 9/22

(54) **DEVICE AND METHOD FOR ELIMINATING VOLATILE ORGANIC COMPOUNDS AND MICROORGANISMS FROM INDOOR AIR**

(30) Priority: 26.02.2024 ES 202430137
(71) Applicant: Molina Herrero, Mercedes, 28043 Madrid (ES)
(72) Inventor: Molina Herrero, Mercedes, 28043 Madrid (ES)
(74) Representative: Isern Patentes y Marcas S.L.
(86) International application number: PCT/ES2025/070100
(87) International publication number: WO 2025/181413

(57) **Abstract**

The device comprises a casing (1), with the following three consecutive chambers (2, 3, 4): - first chamber (2), with a fan (5) having blades (6) coated with SiO₂ nanocrystals; - second chamber (3), with: obstacles (7) to create turbulence, and coated with SiO₂ nanocrystals; ultraviolet light lamps (8); and a photocatalyst solution permeating the inner walls (11) of the second chamber (3); and - third chamber (4), with an ozone-free needlepoint bipolar air ionisation system, and from which purified air is expelled. The invention makes it possible to optimise the elimination of harmful agents in indoor air and to avoid the production of harmful agents, all with less noise.

## Description

### Field of the invention

The present invention is applicable in the air purification sector. In particular, the invention relates to a device, and a method, for treating air to eliminate volatile organic compounds and microorganisms in indoor air.

### Background of the invention

Air can carry a variety of pollutants harmful to health, such as: microorganisms, which cause airborne diseases; allergens; and volatile organic compounds (VOCs); as well as biological or chemical particles, for example, from biomedical waste handled in medical facilities, in laboratories, etc.

These pollutants in indoor air are usually controlled and eliminated by using filters in heating ventilation and air conditioning systems, such as High Efficiency Particulate Air (HEPA) filters. HEPA filters can prevent the spread of bacteria and viruses through the air, for example, in premises with biomedical applications, and therefore prevent infections in said premises. Top-rated HEPA filters have 99.995% efficiency, ensuring a high level of protection against airborne diseases. Particles are trapped in the fibres that make up these filters. Microorganisms, however, are not eliminated. On the contrary, they accumulate and create colonies on the filter, infecting and spreading diseases through the ventilation system. Furthermore, the use of HEPA filters in ventilation systems causes significant pressure drops, reducing their performance and efficiency.

A known alternative solution for indoor air treatment involves the use of ultraviolet (UV) light emissions to eliminate bacteria in the air by exposing said air to a plurality of ultraviolet (UV) lamps. Similarly, ultraviolet (UV) lamps can be used to irradiate a titanium dioxide (TiO₂) matrix to eliminate airborne polluting particles as they pass through said irradiated matrix.

Another known alternative solution for the treatment of polluted air is to expose said air to a plasma discharge. However, as by-products of said discharge, ozone (O₃) and nitrogen oxide (NO) are generated, which are hazardous chemicals harmful to human health if they reach the indoor environment of the premises where the air treatment device or appliance is located.

Air treatment devices are also known which combine the aforementioned solutions, in other words, they first perform a first step of treating the polluted air flow coming from premises with atmospheric plasma treatment means, where part of the volatile organic compounds (VOCs) and microorganisms contained in the air flow are eliminated, and then a second step of treating said air flow in photocatalyst treatment means is performed, where the remaining volatile organic compounds (VOCs) and microorganisms contained in the air flow are eliminated, as well as the by-products of the plasma discharged in the plasma treatment means, which are carried away by the air flow.

All these known solutions have the drawback that they require powerful driving means suitable for inducing the air flow to be treated along the air flow path formed in the device, which passes through the atmospheric plasma treatment means and the photocatalyst treatment means. These mechanical driving means, such as fans or compressors, consume a significant amount of energy in addition to the energy required for the operation of the two air treatment means used and they also generate undesirable noise. These systems rely exclusively on the use of chemical agents and ozone-generating elements for their effectiveness.

### Description of the invention

The present invention resolves the aforementioned drawbacks by means of, according to a first aspect, a device for eliminating volatile organic compounds and microorganisms in indoor air, according to independent claim 1. According to a second aspect, the invention relates to a method for eliminating volatile organic compounds and microorganisms in indoor air, which makes use of the preceding device according to independent claim 12. Other optional advantageous features are described in the preferred exemplary embodiment and in the independent claims.

By means of the present invention, harmful environmental agents such as: bacteria, viruses and other pathogens, biological or chemical particles, etc., are eliminated in an optimised way, thanks to the combined application of three technologies (silicon dioxide coating, photocatalytic oxidation, and needlepoint bipolar ionisation) applied in three chambers.

The aim of this technology is to optimise the elimination of harmful agents without producing by-products that are harmful to health, using so-called "low noise" equipment, i.e., generally below 40 dB, usually even below 30 dB. This equipment makes up for the shortcomings in the aforementioned and existing technologies through the application of innovation and technology.

### Brief description of the figures

The previous advantages and features, in addition to others, shall be understood more fully in light of the following detailed description of an exemplary embodiment, with reference to the following figures 1-2, which must be taken by way of illustration and not limitation.
Figure 1 shows a schematic plan view of the eliminating device of the present invention.
Figure 2 shows a detailed, enlarged schematic view of figure 1.

### List of references

- 1: Casing
- 2: First chamber
- 3: Second chamber
- 4: Third chamber
- 5: Fan
- 6: Blades
- 7: Obstacles (VERTICAL)
- 8: Lamps (HORIZONTAL)
- 9: Housings (HORIZONTAL)
- 10: Metal wires
- 11: Walls

### Detailed description of the invention

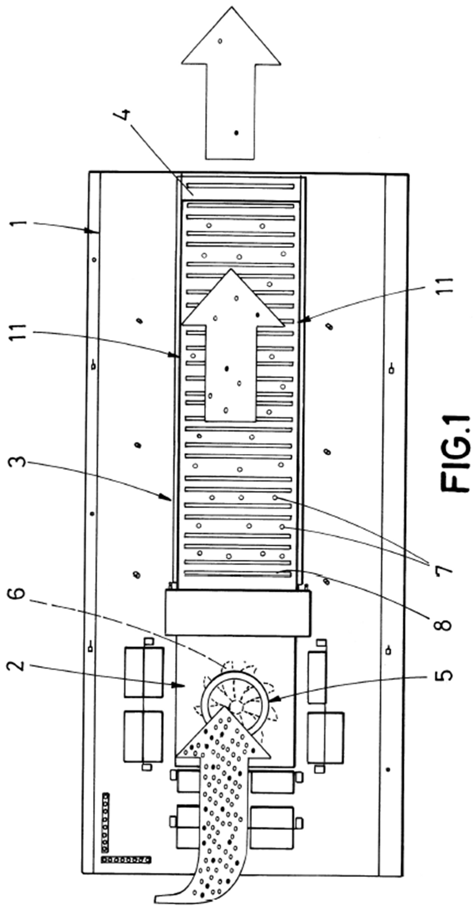

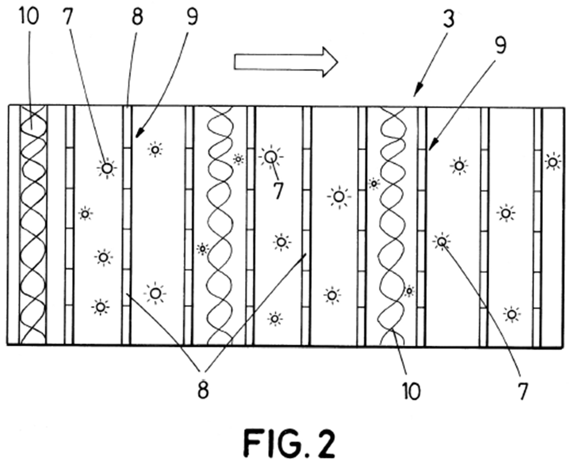

A detailed description of a preferred exemplary embodiment of a device and a method for eliminating volatile organic compounds and microorganisms in indoor air, object of the present invention, is given below with the help of the attached figures 1-2 referred to above.

The device comprises a casing (1), which houses the rest of the components, and which allows for easy installation in homes, commercial premises, catering establishments, hospital facilities, etc.

Inside the casing (1), there are three chambers (2, 3, 4). Of the three chambers (2, 3, 4), a first chamber (2) includes a fan (5), preferably with a low noise level, which has blades (6) coated with silicon dioxide (SiO₂) nanocrystals. The described fan (5) has a dual function: on the one hand, to force polluted air into the interior of the device - in particular the casing (1) - in order to capture and therefore remove said polluted air and, on the other hand, to act as a first means for eliminating undesirable compounds (volatile organic compounds and microorganisms) by means of silicon dioxide.

In the first chamber (2), through the silicon dioxide nanocrystals, both chemical and biological harmful agents are initially removed by combined mechanical and chemical action, instead of the traditional purely chemical elimination. The silicon dioxide is modified to acquire antimicrobial properties and is therefore called "functionalised silicon dioxide", to which a coating of an antimicrobial compound, such as quaternary ammoniums and functionally equivalent compounds, is bonded, the operation of which is explained below.

SiO₂ nanocrystals can form an ultra-thin coating on surfaces, creating an almost invisible protective barrier. Although pure SiO₂ is not antimicrobial, its structure allows other agents to adhere to its surface. Therefore, if SiO₂ nanocrystals are modified with adhered antimicrobial compounds, they can act as a hostile surface for microorganisms.

When SiO₂ nanocrystals are functionalised, as described above, with quaternary ammonium groups or other positively charged nitrogen molecules, an electrostatic attraction is created by the positively charged nitrogen towards the negatively charged cell membranes of the microorganisms, which leads the microorganisms to strike the silicon dioxide nanocrystals, so that the nanocrystals pierce the cell membrane of the microorganisms, disrupting the lipid bilayer and causing cell lysis, which destroys the microorganisms.

The functionalisation of silicon dioxide crystals with positive nitrogen compounds, as explained above, has the following advantages:
- Long-lasting effect: Unlike liquid disinfectants, a coating functionalised with SiO₂ nanocrystals can remain active for long periods.
- No reactivation required: It works without the need for UV light or external agents.
- Broad spectrum: It can act against bacteria, fungi and some viruses.
- It is not mutagenic, unlike other substances such as triclosan.

The fan (5) moves the polluted air towards a second chamber (3) containing obstacles (7) to create turbulence to the polluted air flow. The obstacles (7) are permeated with silicon dioxide (SiO₂) nanocrystals with fungicidal, antibacterial and antiviral properties by means of the coating described above. The obstacles (7) may be in the form of tubes, usually cylindrical, made of metal, and preferably arranged vertically. The turbulence increases the contact of the polluted air with the silicon dioxide, resulting in a dual effect: on the one hand, it increases the residence time of the microorganisms in the second chamber (3) and therefore the chances of being neutralised by the silicon dioxide nanocrystals and the ultraviolet light, while on the other hand, the turbulence facilitates the aforementioned electrostatic attraction between the silicon dioxide nanocrystals and the microorganisms.

The choice of nanocrystals for silicon dioxide coatings allows for high adhesion and integration with the coated substrate, without interfering with its function, and doing so invisibly.

The second chamber (3) also includes ultraviolet light lamps (8) with germicidal properties. The lamps (8) are preferably supported in ultraviolet light-translucent housings (9), for example, made of quartz or borosilicate, which may be tubular in shape, depending on the lamps. The lamps (8) and, where appropriate, the housings (9), may be distributed transversely across the second chamber (3). The device may further include control means (not shown), for example, housed in the casing (1), for controlling the operation of the lamps (8), for example, by adapting operating characteristics of the lamps (8), such as voltage. The control means may additionally be configured to control the operation of the fan (5), for example, the intensity of the fan (5). Moreover, the control means may include sensors (not shown) for detecting and preferably quantifying particular predetermined compounds, such as: humidity; temperature; formaldehyde; airborne particles of maximum micron size equal to or greater than a predetermined value, such as PM 1.0, PM 2.5, PM 4.0, PM 10.0); total volatile organic compounds (TVOC), ozone (O₃), and NO₂.

The control means may additionally include one or more CO₂ concentration meters (not shown), which are indicators of the concentration of microorganisms. The control means may be configured to control the voltage of the lamps (8) and/or the intensity of the fan (5) based on the measurements from the sensors and the concentration meters. Determining the concentrations of microorganisms based on the measurements of CO₂ and, where appropriate, controlling the voltage of the lamps (8) and/or the intensity of the fan (5) can be carried out according to a preferred embodiment by means of artificial intelligence techniques, such as machine learning and deep learning.

Inserted between the housings (9) for the lamps (8), there is, preferably horizontally across the second chamber (3), such as from side to side, a network of metal wires (10), especially made of noble metals, such as gold, silver, platinum, etc., to provide a germicidal effect.

In the second chamber (3), the housings (9) of the lamps (8) and/or the walls (11) inside the second chamber (3) itself and/or the metal wires (10) are permeated with a photocatalyst solution, for example, based on titanium dioxide, which may be supplemented with one or more additional catalysts, in order to produce photocatalytic oxidation.

At the outlet of the second chamber (3), there is a third chamber (4), to which the polluted air is transferred from the second chamber (3), and which houses an ozone-free needlepoint bipolar air ionisation system (not shown) that, in addition to eliminating the remaining harmful agents (chemical and biological) by ionising the resulting air, it charges the air with ions, sanitising the surfaces where they are deposited, before expelling purified air to the outside of the device, in particular the casing (1). In this way, the ions in the expelled air disinfect the surfaces on which they land, as well as neutralise biological agents (microorganisms, bacteria) and VOCs (volatile organic compounds) by colliding with them. This system, and its associated operating method, ensures the elimination of agents harmful to health in the environment (chemical agents, biological agents, viruses).

## Claims

1. A device for eliminating volatile organic compounds and microorganisms in indoor air, **characterised in that** it comprises:
- a casing (1), housing: a first chamber (2); a second chamber (3) downstream of the first chamber (2); and a third chamber (4) downstream of the second chamber (3);
- a fan (5), housed in the first chamber (2), to capture polluted air, and comprising blades (6) coated with silicon dioxide (SiO₂) nanocrystals, in turn coated with a positively charged nitrogen antimicrobial compound to electrostatically attract and destroy the microorganisms;
- obstacles (7) located in the second chamber (3), to create turbulence in the polluted air received from the first chamber (2), and which are permeated with silicon dioxide (SiO₂) nanocrystals, in turn coated with a positively charged nitrogen antimicrobial compound, to electrostatically attract and destroy the microorganisms;
- ultraviolet light lamps (8) located in the second chamber (3);
- a photocatalyst solution that permeates inner walls (11) of the second chamber (3); and
- an ozone-free needlepoint bipolar air ionisation system located in the third chamber (4), from which purified air is expelled.

2. The device according to claim 1, wherein the obstacles (7) are in the form of metal tubes, preferably arranged vertically.

3. The device according to any of claims 1-2, additionally comprising housings (9) for supporting the lamps (8) of the second chamber (3), which are translucent to ultraviolet light,

4. The device according to claim 3, wherein the housings (9) are distributed transversally across the second chamber (3).

5. The device according to any of claims 3-4, further comprising a network of metal wires (10), inserted between the housings (9) for the lamps (8).

6. The device according to claim 5, wherein the metal wires (10) of the network are arranged horizontally across the second chamber (3).

7. The device according to any of claims 5-6, wherein the metal wires (10) are made of noble metals.

8. The device according to any of claims 1-7, wherein the fan (5) is a fan (5) with a noise level of less than 40 dB.

9. The device according to any of claims 1-8, further comprising control means for controlling the operation of the lamps (8) and/or the fan (5).

10. The device according to claim 9, wherein the control means comprise:
- at least one sensor for detecting presence and, where appropriate, concentration of predetermined compounds; and
- one or more CO₂ concentration meters to estimate the concentration of microorganisms, the control means being configured to control the operation of the fan (5) and/or the lamps (8) from the sensors and the CO₂ meter(s).

11. The device according to claim 10, wherein the at least one sensor is selected from one or more of the following:
- humidity sensor;
- temperature sensor; formaldehyde sensor;
- sensor of airborne particles of maximum size equal to or greater than a predetermined value;
- total volatile organic compounds (TVOC);
- ozone (O₃), and
- NO₂.

12. The device according to any of claims 1-11, wherein the housings (9) of the lamps and/or the metal wires (10) are also permeated with the photocatalyst solution.

13. The device according to any of claims 1-12, wherein the photocatalyst solution comprises one or more catalysts including titanium dioxide.

14. The device according to any of claims 1-13, wherein the positively charged nitrogen antimicrobial compound comprises quaternary ammoniums.

15. A method for eliminating volatile organic compounds and microorganisms in indoor air, using the device described in any of claims 1-14, **characterised in that** it comprises:
- forcing polluted air into the casing (1) through the fans (5) of the first chamber (2);
- treating the polluted air by means of the silicon dioxide nanocrystals that coat the blades (6) of the fan (5), so that the positively charged nitrogen of the antimicrobial compound that coats the silicon dioxide nanocrystals attracts the negatively charged cytoplasmic membranes of the microorganisms, so that the membranes strike the silicon dioxide nanocrystals, which tear said membranes, destroying the microorganisms;
- transferring the polluted air from the first chamber (2) to the second chamber (3), so that the silicon dioxide nanocrystals attract the microorganisms, the microorganisms striking the silicon dioxide nanocrystals, which tear the cytoplasmic membranes of the microorganisms;
- causing turbulence in the polluted air in the second chamber (3) by means of the obstacles (7);
- treating the polluted air in the second chamber (3) by means of the silicon dioxide nanocrystals permeating the obstacles (7);
- treating the polluted air in the second chamber (3) with ultraviolet light from the lamps (8);
- treating the polluted air in the second chamber (3) with the photocatalyst solution;
- transferring the polluted air from the second chamber (3) to the third chamber (4); and
- treating the polluted air in the third chamber (4) with the ozone-free needlepoint bipolar air ionisation system.

16. The method according to claim 15, wherein the turbulence caused by the obstacles (7), on the one hand, increases the residence time of the microorganisms in the second chamber (3) and, on the other hand, facilitates the electrostatic attraction between the silicon dioxide nanocrystals and the microorganisms.

17. The method according to any of claims 15-16, additionally including controlling the lamps (8) by varying some of their characteristics, such as voltage.

18. The method according to any of claims 16 and 17, including controlling the fan (5) by varying some of its characteristics, such as power.

19. The method according to any of claims 17 and 18, wherein the controlling step includes:
- taking measurements with the sensors for predetermined compounds, and with the CO₂ concentration meter;
- determining the presence and, where appropriate, the concentration of the predetermined compounds, as well as the concentration of CO₂, based on the measurements; and modifying the operation of the fan (5) and/or the lamps (8) based on the concentration of the predetermined compounds and the concentration of CO₂.

20. The method according to claim 19, wherein the modification of the operation of the fan (5) and/or the lamps (8) based on the concentrations comprises the use of artificial intelligence techniques, such as machine learning and deep learning.
